# EUROPEAN PATENT APPLICATION

(11) **EP 1 367 381 A1**
(43) Date of publication of application: **03.12.2003**
(21) Application number: 02425337.9
(22) Date of filing: 28.05.2002
(51) Int. Cl.: G01N 1/30

(54) **Composition for the preparation of histological, autopsical, cytological samples**

(71) Applicant: Imberg S.r.l., 24057 Martinengo (BG) (IT)
(72) Inventor: Mosconi, Gianfilippo, 60039 Staffolo (AN) (IT); Bianchi, Monica, 24060 Casazza (BG) (IT)
(74) Representative: Trupiano, Federica

(57) **Abstract**

A composition based on C₅-C₁₂ alkyl and at least one aliphatic alcohol, used for the preparation of histological, autopsical and cytological specimens (samples) that are to undergo examination, said composition being designed to be used instead of different reagents that have up to the present been used according to rigidly fixed sequences.

## Description

### SUBJECT OF THE INVENTION

The subject of the present invention is a composition and its use for the preparation of histological, autopsical and cytological specimens (samples) and the like that are to undergo examination.

### STATE OF THE ART

As is known, the analysis of histological, autopsical and cytological specimens (samples) and the like is of fundamental importance in modern medicine, in so far as it not only enables exact and often significantly early diagnosis of an extremely large number of illnesses, but also enables complex studies to be conducted, which contribute to progress in the sector. Of course, the specimens (samples) to be analysed must be treated and prepared according to precise protocols, in order to guarantee an optimal preservation, reproducibility of analysis, and reliability of the results.

At present, in the case of a specimen (sample) taken from a piece coming from an operation or autopsy that has possibly been previously fixed (for example, a tissue specimen (sample)), the first stage of preparation of the specimen (sample) for examination envisages a so-called processing step and a possible fixation step, which has the purpose of preparing the piece for embedding in a means having a consistency such as to bestow upon it the rigidity necessary for cutting with blades. The embedding material that currently best satisfies these requirements is paraffin, which is, however, not mixable with the majority of the fixatives used. For this reason, for the tissue to be embedded in paraffin and then cut according to the requirements, it must undergo various treatments, all of which come under the processing step, which may be summarized as follows:
- Dehydration step. This envisages the replacement of the water contained in the specimen (sample) with anhydrous reagents,
- Clarification step. This envisages the replacement of the anhydrous reagent with a component (clarifying means) that is mixable both with the anhydrous reagent and with the embedding means,
- Impregnation step. This envisages the replacement of the clarifying means with the impregnation means (or embedding means).

The first step envisages the removal of the aqueous reagents used in the initial step of fixation of the specimen (sample) that has just been taken. Numerous dehydrating agents can be used, but preferably ethyl alcohol is used in increasing concentrations, i.e., the specimen (sample) is first treated with 70% ethyl alcohol, then with 95% ethyl alcohol, and then again with 100% ethyl alcohol. Since the dehydrating agents most commonly used are not soluble in paraffin (the means in which the specimen (sample) must be embedded), they must therefore be replaced with a component that is soluble both in the dehydrating agent and in the impregnating agent. In this step, xylene (or xylol, dimethylbenzene) is commonly used, which is an aromatic hydrocarbon consisting of a mixture of ortho-, meta-, and para-isomers, in the form of an inflammable, highly volatile and potentially very toxic liquid. The potential toxicity of xylene represents a constant problem for those who are called upon to make use of it. The non-specific effect on the central nervous system manifests itself first with a sense of nausea and gastro-intestinal disorders and, if the intoxication continues, this is followed by dizziness, torpidity and vomiting. Inhalation causes irritation of the respiratory mucosa and probable pulmonary edema. In the case of chronic exposure, alterations have been observed in the central nervous system, the leukocyte formula, and cardiac functionality, but above all a potential carcinogenicity.

Once again according to the known art, the processing step is generally followed by a step of embedding in paraffin, a cutting step, and a staining step. The latter step further envisages the elimination of the paraffin (which simply had the function of substrate for cutting) by means of treatment with xylene, a rehydration step with alcohols of variable titre (100%, 95%, 70%), the staining proper of the specimen (sample), another dehydration stage (70%, 95%, 100%), and finally again treatment with xylene to eliminate the dehydrating agent.

Also in the case of analysis of cytological specimens (samples), where embedding in paraffin is not envisaged or necessary, there is instead required the repeated use of xylene given that, also in these cases, the specimen (sample) undergoes numerous steps of hydration and dehydration.

It thus appears evident that the repeated use of xylene within a sequence to be used for the preparation of histological and/or autopsical specimens (samples) to be subjected to examination involves a certain risk for the operator, also in the cases where the majority of the sequences occur according to an automated system. In fact, the contact with the toxic reagent is in any case envisaged, and its replacement with a component that presents similar characteristics but much lower toxicity would certainly be desirable.

### OBJECTS OF THE INVENTION

An object of the present invention is to provide a composition for the preparation of histological, autopsical and cytological specimens (samples) and the like that is not carcinogenic and that has a low toxicity value.

A further object of the present invention is to provide a composition for the preparation of histological, autopsical and cytological specimens (samples) and the like that will enable numerous steps to be eliminated, which up to the present have been indispensable in the preparation of the specimen (sample) to be examined, as well as, in certain cases, enabling a saving also in terms of the time required for the preparation.

Yet a further object of the present invention is to provide a composition for the preparation of histological, autopsical and cytological specimens (samples) and the like that will replace different reagents, with a consequent diminished risk of confusion and hence of error on the part of the operator.

Another object of the present invention is to provide a method for the preparation of histological, autopsical and cytological specimens (samples) to be examined that is fast, effective and reliable, that does not entail risks for operators, and that ensures an optimal preservation of the specimen (sample), with consequent reproducibility and reliability of the results deriving from the examination of the specimen (sample) itself.

### DESCRIPTION

The above and yet further objects and corresponding advantages, which will emerge more clearly from the ensuing description, are achieved by a composition for the preparation of histological, autopsical and cytological specimens (samples) and the like that are to undergo examination, which comprises at least one C₅-C₁₂ alkyl and at least one aliphatic alcohol.

According to the present invention, the composition comprises octane, isopropyl alcohol, and ethyl alcohol. Said ethyl alcohol is understood as absolute alcohol (99.9%). More particularly, said composition comprises octane between 40 vol% and 60 vol%, isopropyl alcohol between 10 vol% and 30 vol%, and ethyl alcohol between 10 vol% and 30. vol%.

In detail, the composition according to the invention comprises 50 vol% octane, 25 vol% isopropyl alcohol, and 25 vol% ethyl alcohol, and in particular in this case, the quantity of an, aliphatic alcohol is equal to that of the other alcohol and the sum of the amount of alcohol is equal to the amount of C₅-C₁₂ alkyl.

A process for the preparation of the composition according to the invention envisages the mixing of the components up to complete homogeneity.

The aforesaid composition according to the present invention is advantageously used in the preparation of histological, cytological and autopsical specimens (samples) and the like as a replacement for ethyl alcohol of various titre and xylene which, as has already been said, is highly toxic and potentially carcinogenic. The components of the composition according to the invention do not, instead, present high toxicity, and there are no indications of any possible carcinogenicity. In practice, for example, in the case of the preparation of an autopsical and/or histological specimen (sample), the composition according to the invention can be used instead of the sequence of ethyl alcohol of different titre, as well as instead of xylene in all the steps of preparation of the specimen (sample), and it is possible to carry out the step of dehydration and clarification using the composition alone. In particular, for the processing step, from the ensuing tables referring to the processing of so-called "big" specimens (samples), it is possible to highlight the advantage that is obtained from the use of the composition according to the present invention.

In the tables given below, the composition forming the subject of the present invention is indicated as Composition A.

With reference to the attached tables, appearing in Table 1 is the procedure commonly followed for carrying out the operations for processing a specimen (sample) according to the known art, whilst Table 2 presents the same procedure using the composition A.

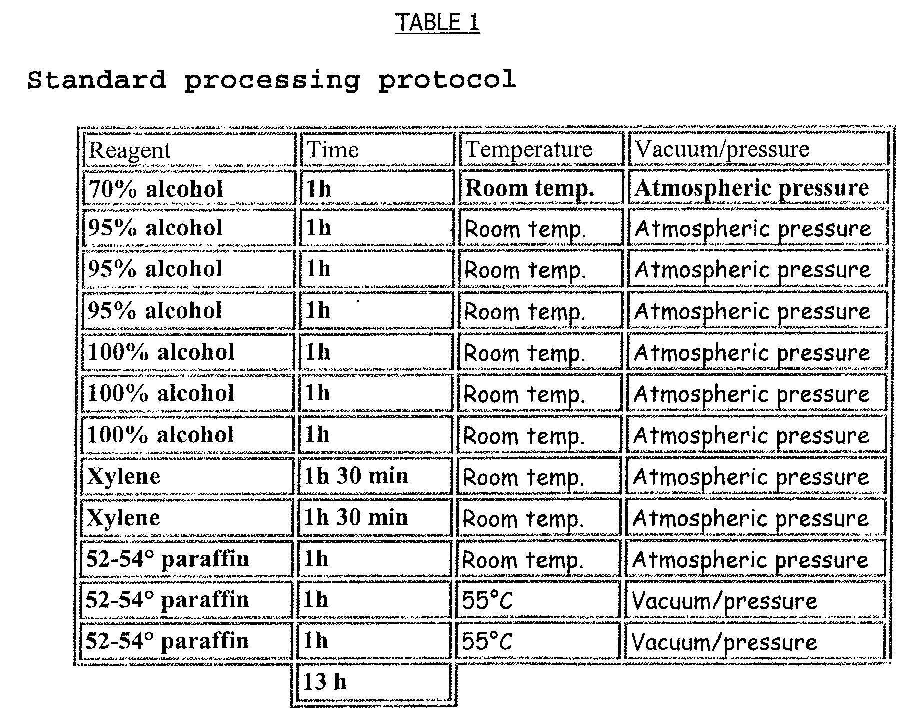

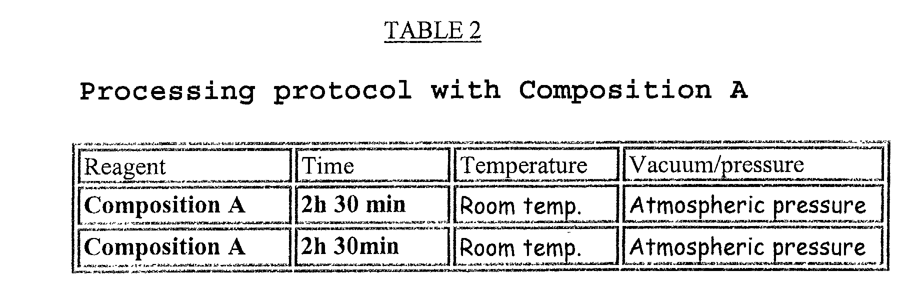

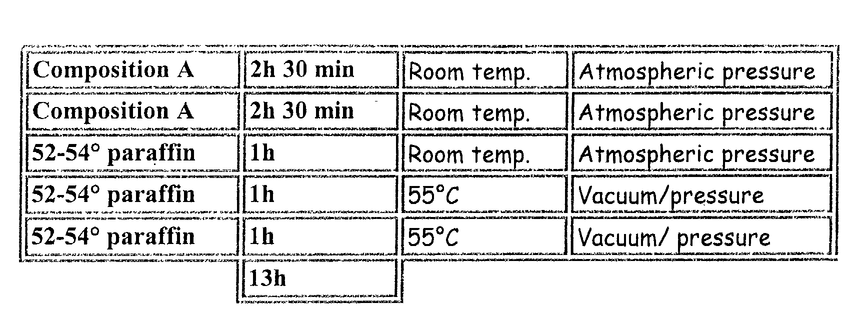

As may be seen, even though the total time necessary for the preparation of the specimens (samples) has remained unvaried, the treatments in the solutions of alcohol of different degree and the subsequent treatments in xylene are replaced by treatments in the composition A alone.

The composition according to the present invention thus enables the step of dehydration and clarification to be carried out with a single component. In this way,

it is possible to carry out the processing step with a lower number of treatments using a non-toxic and non-carcinogenic substance, as compared to xylene, which, instead, is necessary if the procedure according to the known art is followed.

Again with reference to the process of preparation of histological and autopsical specimens (samples) to be examined, as has already been said, it is necessary to embed the piece in a substrate that enables it to be cut into sections suitable for examination. The embedding means most commonly used is paraffin, both because

it is advantageous from the economic standpoint and because it is easy to manipulate and enables very thin tissue sections to be obtained.

Described below are the subsequent steps for embedding the piece in paraffin, cutting it into the desired sections, eliminating the paraffin that provides the substrate, and the procedure for staining the specimen (sample) to be analysed.

Also for these subsequent steps, the use of the composition forming the subject of the invention is of fundamental importance in the replacement of the steps of treatment of the specimen (sample) with alcohol of various titre and with xylene.

### - Embedding

Once processed, the specimens (samples) to be examined are embedded in paraffin blocks at a melting point of 56-58°C using steel moulds.

### - Cutting

Cutting of the sections is performed using microtomes to obtain sections of 3-5 µm in thickness. These slices of tissue are gathered using brushes and laid in a bath containing water at 35°C to enable their distension.

Next, the sections are gathered on slides.

The slides are then set to dry in a stove for variable periods of time and at variable temperatures according to the staining protocol that it is intended to apply subsequently.

### - Step of elimination of the paraffin

In order for a tissue section to be stained, it is necessary to eliminate the layer of paraffin in which it is embedded to enable the different reagents to act directly on the tissue.

For this reason, the protocol given below is followed, which shows, in Table 3, the procedure commonly used according to the known art, whilst Table 4 shows the procedure that envisages the use of the Composition A according to the invention.

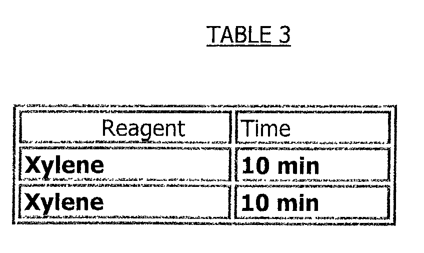

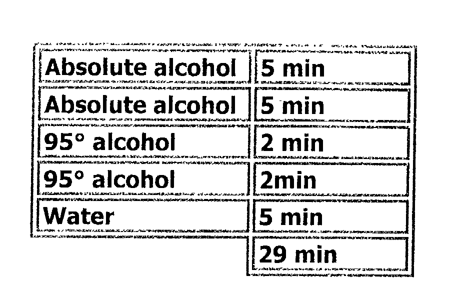

The composition according to the invention, by replacing the step of treatment with alcohols and xylene, enables the step of elimination of the paraffin to be carried out in just two steps:

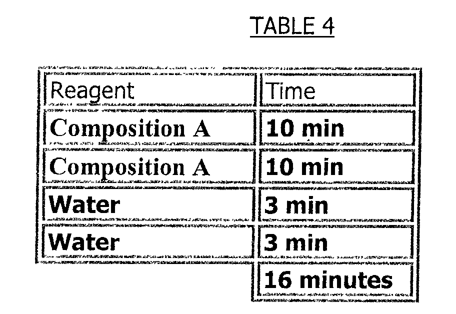

### - Diaphanization

After completion of any staining protocol, the section must be dehydrated again to apply the slide using a resinous mounting means. This enables better preservation of the section over time.

Usually, to carry out this additional step, the protocol appearing below is applied, which illustrates the procedure as performed according to the known art, in comparison with the steps appearing in Table 6, which envisage the use of the Composition A of the present invention.

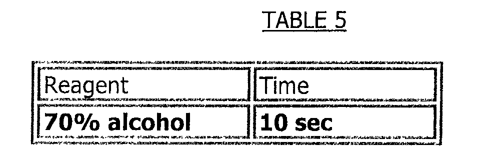

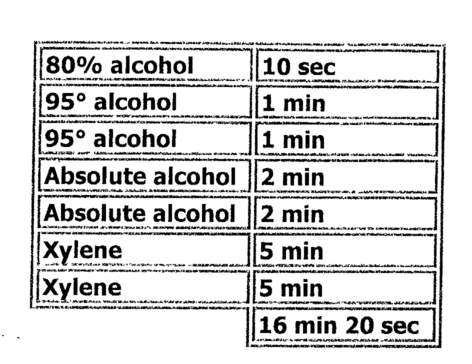

The composition according to the present invention, by replacing alcohols and xylene, enables also the step of diaphanization to be carried out in just two steps:

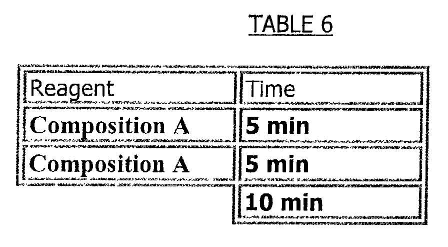

In addition to what is described above, the composition according to the invention can also be used in any histological staining protocol that entails intermediate treatments using alcohols at different percentages that are to be combined with possible treatment with xylene, in some cases maintaining the time of the treatment constant but guaranteeing a clear improvement as regards the manual convenience of the method and the ease of execution.

Tables 7 and 8 give, respectively, the procedure of haematoxylin/eosin staining according to the known art and the procedure using the composition according to the present invention.

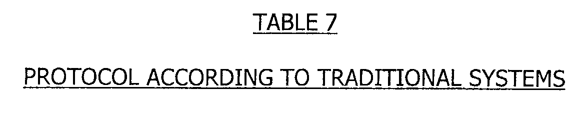

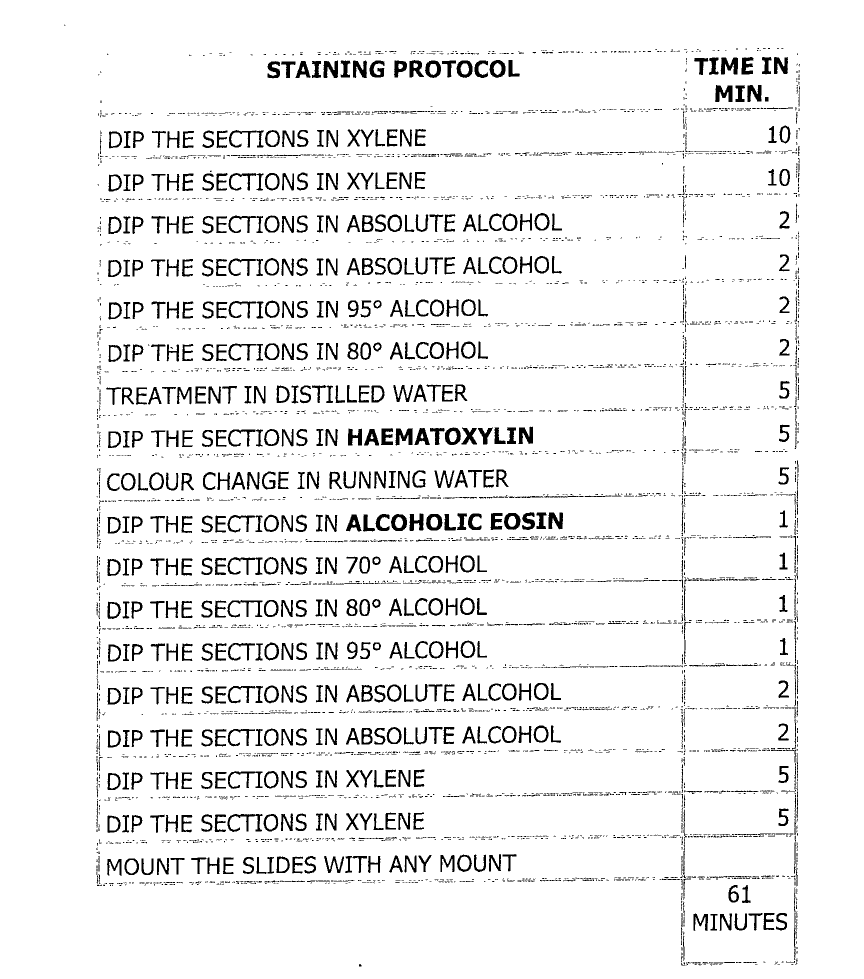

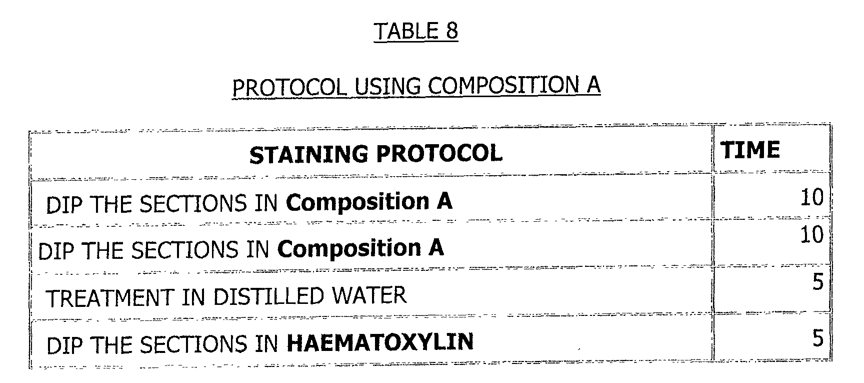

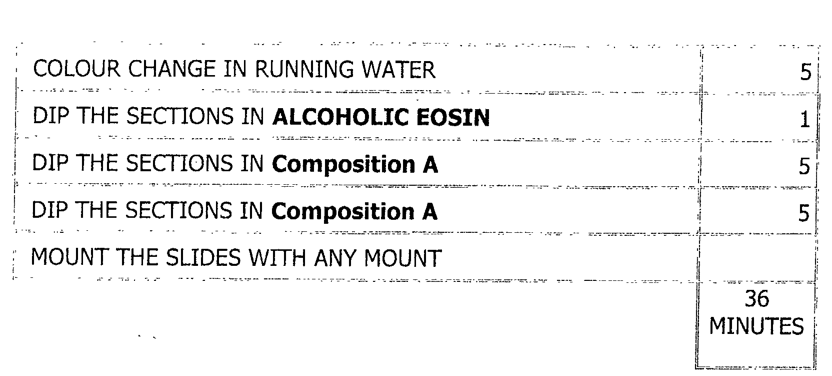

In this case, in addition to the advantages already extensively described herein, there emerges also a considerable saving in time when the procedure is performed using the composition according to the invention.

### - Cytological specimens (samples)

The composition according to the invention can be advantageously used also for the preparation of cytological specimens (samples), i.e., ones fixed for example in alcohol and ether and not embedded in paraffin or other embedding agent, such as urine samples, Pap test, sputum and other liquid specimens (samples), or specimens (samples) of another nature.

The composition according to the invention replaces:
1. All the washings in alcohols of different concentration, whilst maintaining constant the total time of the washings, or possibly reducing said time, according to the particular cases.
2. The process of final diaphanization.

Tables 9 and 10 provide examples of PAPANICOLAOU staining both according to the known art and using the composition according to the present invention.

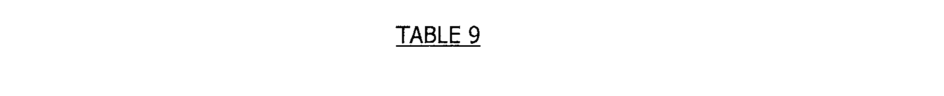

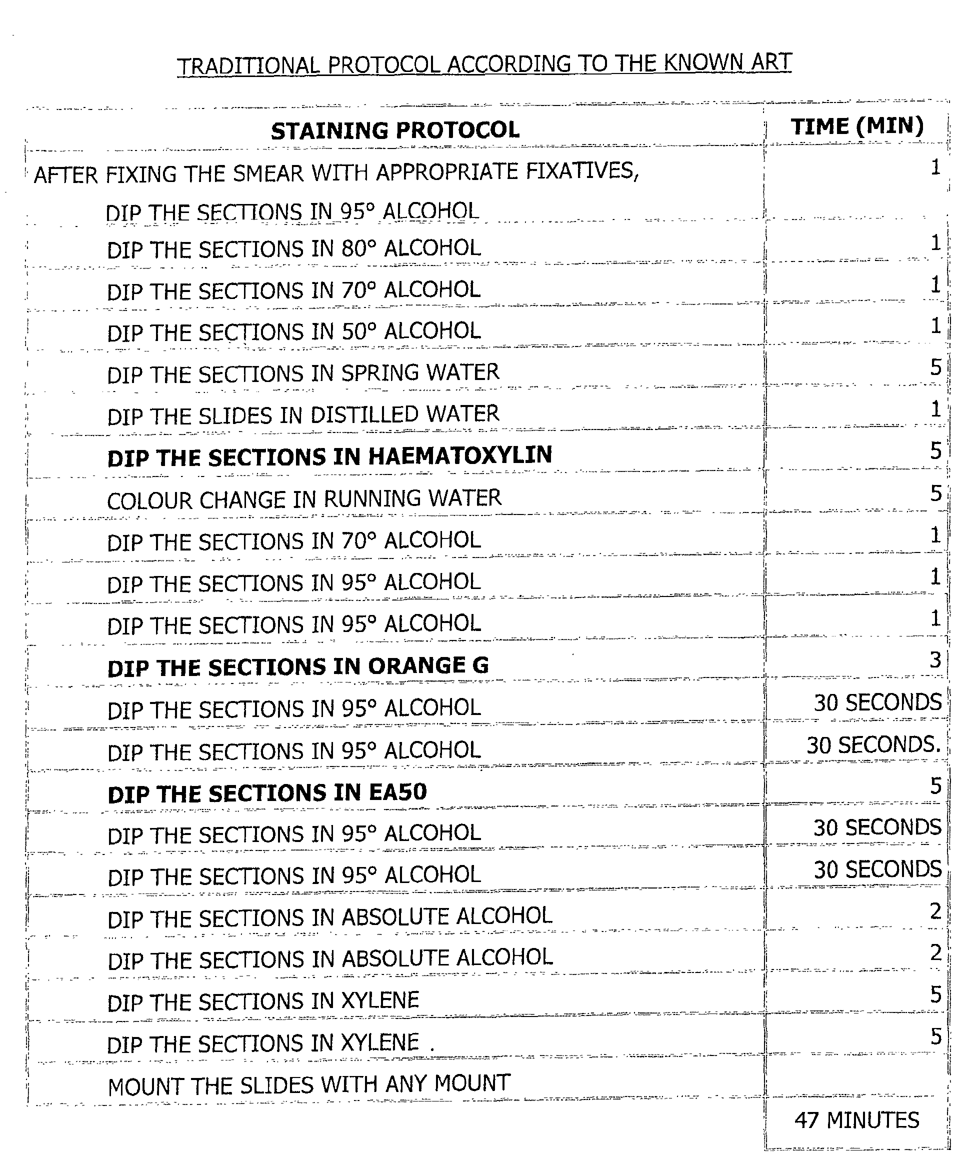

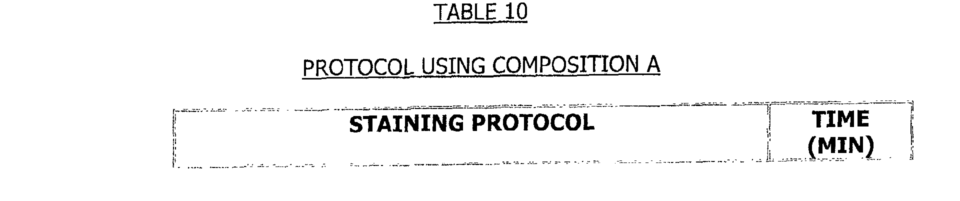

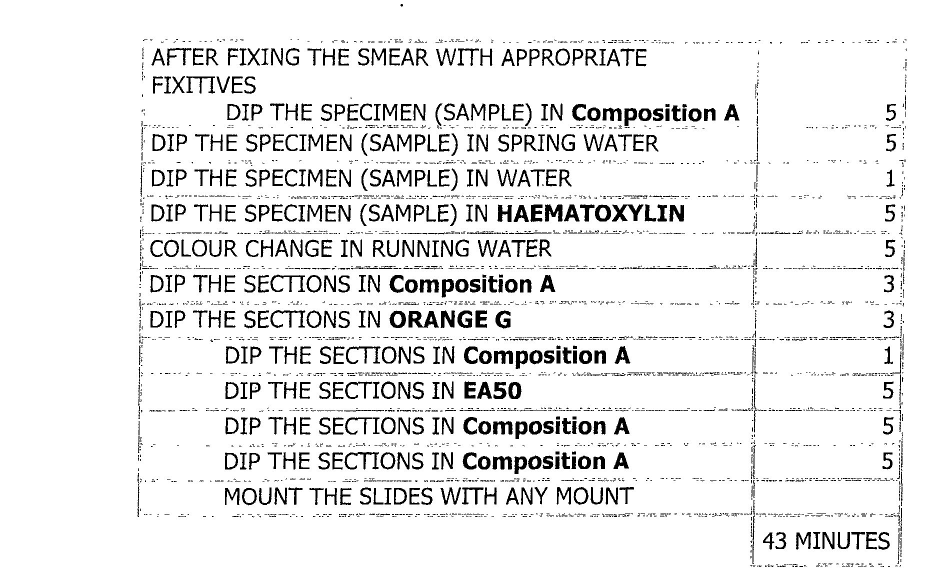

In this case, even though no significant saving in time is noted, the saving in terms of fewer steps required and smaller number of reagents used appears evident.

As has already been said above, the composition according to the present invention enables the various steps of preparation of the autopsical, histological and cytological specimens (samples) to be carried out, by replacing completely xylene and alcohols and thus preventing the repeated use of different reagents at different times and according to rigidly fixed sequences. It is moreover evident that the operator, when obliged to use a lot of reagents different from one another according to a pre-set order, may easily become confused and may commit errors that might have repercussions on the final result of the analysis.

The composition according to the present invention moreover enables reduction of the times involved in the step of elimination of the paraffin in the corresponding stage, thanks to the considerable affinity between the composition itself, the paraffin, and the other reagents used.

The said composition does not alter the morphological characteristics and the antigenic expression of the tissue upon which it bestows brilliance. In addition, it does not present any interaction with plastic material and can be advantageously used also in contact with materials commonly employed in the laboratory that are other than glass.

In the case where the preparation is made on a suitable specimen (sample), the composition which has been used and which comes from the various steps of the procedure can be recovered and, after undergoing treatments of adequate purification, can possibly be re-used. Of course, the possible recycling after purification must be compatible with the type of potential contamination of the composition, which depends chiefly upon the illnesses found in the subjects from whom the specimen (sample) - which has been prepared and which has come into contact with the composition according to the invention - has been taken.

## Claims

1. A composition comprising at least one C₅-C₁₂ alkyl and at least one aliphatic alcohol.

2. The composition according to Claim 1, comprising octane, isopropyl alcohol, and ethyl alcohol.

3. The composition according to Claims 1 and 2, **characterized in that** it comprises octane between 40 vol% and 60 vol%, isopropyl alcohol between 10 vol% and 30 vol%, and ethyl alcohol between 10 vol% and 30 vol%.

4. The composition according to Claims 1 to 3, **characterized in that** it comprises octane at 50 vol%, isopropyl alcohol at 25 vol% and ethyl alcohol at 25 vol%.

5. Use of the composition according to Claims 1 to 4 for the preparation of histological, autopsical and cytological specimens (samples) that are to undergo examination.

6. A method for the preparation of histological, autopsical and cytological specimens (samples) which comprises the treatment of said specimen (sample) with the composition according to Claims 1 to 4.

7. A process for the preparation of histological, autopsical and cytological specimens (samples) that are to undergo examination, which comprises the treatment of said specimens (samples) with the composition according to Claims 1 to 4.

8. Histological, autopsical and cytological specimens (samples) that are to undergo examination prepared using the composition according to Claims 1 to 4.

9. A method for the examination of histological, autopsical and cytological specimens, (samples) which comprises the treatment of said specimens (samples) with the composition according to Claims 1 to 4.
